# EUROPÄISCHE PATENTSCHRIFT

(11) **EP 1 629 810 B1**
(45) Veröffentlichungstag und Bekanntmachung des Hinweises auf die Patenterteilung: **15.04.2009**
(21) Anmeldenummer: 05015763.5
(22) Anmeldetag: 20.07.2005
(51) Int. Cl.: A61F 13/02

(54) **Folienlaminatpflaster und Verfahren zu dessen Herstellung**
Laminated plaster and method of production
Pansement stratifié et méthode de production

(30) Priorität: 16.08.2004 DE 102004039679
(43) Veröffentlichungstag der Anmeldung: 01.03.2006
(73) Patentinhaber: BSN Medical GmbH, 20253 Hamburg (DE)
(72) Erfinder: Götz, Gabriela, 22395 Hamburg (DE); Mayan, Robert, Dr., 21614 Buxtehude (DE)
(74) Vertreter: UEXKÜLL & STOLBERG

(56) Entgegenhaltungen:
- WO-A-20/05051259
- US-A- 4 619 253
- US-A1- 2004 059 273
- US-B1- 6 225 522

## Beschreibung

Die vorliegende Erfindung betrifft ein Folienlaminatpflaster mit einer Polymerfolie, auf deren einer Seite eine abziehbare Stützfolie vorgesehen ist und deren andere Seite mit einer hautverträglichen Klebeschicht versehen ist, die wiederum von einer abziehbaren Schutzabdeckung abgedeckt ist, wobei auf die Stützfolie eine mit den Fingern greifbare Abziehhilfe zum Abziehen der Stützfolie von der Polymerfolie aufgeklebt ist, wobei die Abziehhilfe aus Klebeband gebildet ist. Die Erfindung betrifft ferner ein Verfahren zur Herstellung eines solchen Folienlaminatpflasters.

Folienlaminatpflaster oder -verbände werden seit längerem im medizinischen Bereich eingesetzt. Im applizierten Zustand haftet eine dünne Polymerfolie mit einer Klebeschicht an der Haut. Ein wichtiges Merkmal derartiger Pflaster oder Verbände ist deren erwünschte Eigenschaft, dass sie zwar für Bakterien und flüssiges Wasser undurchlässig sein sollen, während die Polymerfolie für Luft und Wasserdampf durchlässig sein soll, damit die Wunde einerseits geschützt ist, andererseits aber Luftzugang hat und an der Wunde entstehender Wasserdampf entweichen kann. Diese Eigenschaften lassen sich bei einer homogenen Polymerfolie nur realisieren, wenn diese extrem dünn ist.

Die mit Klebstoff beschichtete Seite der Polymerfolie muss vor der Applikation mit einer Schutzabdeckung bedeckt sein, damit die Klebstoffschicht nach der Herstellung des Pflasters unbeschädigt bleibt. Vor der Applikation des Pflasters wird die Schutzabdeckung abgezogen. Die dann zurückbleibende extrem dünne Polymerfolie ist aber sehr schwer zu handhaben. Es wurde daher bereits vorgeschlagen, die Polymerfolie auf der der Klebeschicht gegenüberliegenden Seite mit einer Stützfolie zu versehen, die erst nach der Applikation an der Wundstelle von der Polymerfolie abgezogen wird und die hinreichend dick ist, um eine sichere und einfache Handhabung des Folienlaminatpflasters zu ermöglichen.

Ein solches Folienlaminatpflaster mit einer Stützfolie, einer Polymerfolie mit Klebeschicht, die von einer Schutzabdeckung abgedeckt ist, ist z.B. in DE 40 26 755 A1 beschrieben. Das dort beschriebene Folienlaminatpflaster, das die Merkmale des Oberbegriffs von Patentanspruch 1 aufweist, hat eine dünne Polymerfolie mit einer Klebstoffbeschichtung auf einer Seite, auf deren gegenüberliegenden Seite eine Stützfolie, z.B. eine Polyethylenfolie mit etwa 80 µm Dicke aufgeklebt ist. Polymerfolie und Stützfolie haben die gleiche Größe, d.h. die Folien überdecken sich vollflächig. Die Klebeschicht an der Polymerfolie wird von einer Schutzabdeckung abgedeckt, die klebstoffabweisende Eigenschaften hat und die aus zwei Teilen besteht, die sich entlang eines streifenförmigen Bereichs überlappen, so dass zumindest der überlappende Randbereich des einen Schutzabdeckungsteils leicht angehoben und dieser Schutzabdeckungsteil leicht abgezogen werden kann. Nach Abziehen des anderen Schutzabdeckungsteils und nach Aufbringen des Verbunds aus Polymerfolie und Stützfolie auf die Wundstelle, wird die Stützfolie von der Polymerfolie abgezogen. Zu diesem Zweck sind Abziehhilfen mit einer Klebstoffschicht auf der Stützfolie aufgeklebt, wobei die Abziehhilfen Folienstreifen sind, die an zwei gegenüberliegenden Rändern des Pflasters auf die Stützfolie mit einer Klebstoffschicht aufgeklebt sind. Diese Folienstreifen sind dabei so auf die Stützfolie aufgeklebt, dass sie über die Ränder der Stützfolie vorstehen und so mit den Fingern leicht ergriffen werden können. Als Beispiele für die Folienstreifen ist z.B. eine LDPE-Folie mit etwa 80 µm Dicke angegeben, wobei die Folienstreifen etwa 3 cm breit sind und auf einem Randbereich von etwa 1 cm Breite an der Stützfolie aufgeklebt sind, so dass etwa 2 cm der streifenförmigen Folie überstehen.

Auf Grund der Tatsache, dass die streifenförmigen Folienabziehhilfen an beiden Rändern und über separat aufzubringenden Klebstoff oder mit doppelseitigem Klebeband auf die Stützfolie aufgeklebt werden müssen, hat sich das oben beschriebene Folienlaminatpflaster als in der Herstellung teuer und aufwendig erwiesen. Bei der Herstellung muss insbesondere ein weiterer Fertigungsschritt, nämlich die Aufbringung des Klebstoffs für die Folienabziehilfen durchgeführt werden. Außerdem müssen mehr Einsatzmaterialen bereitgehalten werden, nämlich der weitere Klebstoff zur Befestigung der Folienabziehilfen, der sich von dem Klebstoff für die Polymerfolie unterscheidet.

Ein ähnliches Folienlaminatpflaster ist in DE 43 14 834 C2 beschrieben, dessen Aufbau sich von dem zuvor beschriebenen Folienlaminatpflaster dadurch unterscheidet, dass die Abziehhilfe als Folienstreifen mit einer Klebstoffbahn so auf die Stützfolie aufgeklebt ist, dass der Folienstreifen nicht über den Umfangsrand der Stützfolie hinausragt. Die erwähnten Probleme bei der Herstellung und die daraus resultierenden erhöhten Herstellungskosten sind jedoch unverändert vorhanden.

Aus EP 0 699 127 B1 ist ein Folienlaminatpflaster bekannt, das ebenfalls eine Schichtfolge aus Stützfolie, Polymerfolie, Klebstoffschicht, Schutzabdeckung aufweist. Hier ist eine Abziehhilfe in Form einer auf die Schutzabdeckung aufgeklebten Folie mit einer von der Schutzabdeckung frei abstehenden Lasche vorgesehen. Die Schutzabdeckung hat angrenzend an die Abziehhilfe einen Einschnitt, so dass die Schutzabdeckung durch Ergreifen der Lasche und Abziehen zunächst von der Klebstoffschicht abgezogen werden kann. In dem der Abziehhilfe gegenüberliegenden Rand des Folienlaminatpflasters ist eine Klebebandverbindung vorgesehen, die am äußeren Rand der unteren Schutzabdeckung aufgeklebt ist, um den seitlichen Rand des Folienlaminatverbands herum verläuft und weiter auf den äußeren Rand der oberen Stützfolie aufgeklebt ist. Durch Anheben der Lasche kann so ein Teil der Schutzabdekkung abgezogen werden, woraufhin das Pflaster auf die zu behandelnde Wundstelle aufgelegt wird. Danach wird weiter an der Abziehhilfe gezogen, wodurch durch die Klebebandverbindung am Rand des Pflasters schließlich auch die obere Stützfolie abgezogen werden kann. Bei diesem Folienlaminatpflaster zeigen sich jedoch Probleme bei der Applikation, da die Schutzabdeckung vor dem Aufbringen auf die Wundstelle nicht vollständig entfernt werden kann, da ein Teil der Schutzabdeckung, nämlich diejenige Fläche, die jenseits der Schnittlinie, dem Schutzabdeckungsteil mit Abziehhilfe gegenüberliegend angeordnet ist, erst im letzten Schritt, wenn ein Teil des Pflasters bereits auf der Wundstelle liegt, abgezogen werden kann. Überdies ist dies bekannte Folienlaminatpflaster auch in der Herstellung aufwendig, da neben einer bereits in sich komplizierten Abziehhilfe mit abstehender Lasche auch noch eine Klebebandverbindung zwischen der Schutzabdeckung und der Stützfolie angebracht werden muss.

US 6 225 522 B1 beschreibt ein Folienlaminatpflaster, wobei die Stützfolie die Fläche der Polymerfolie überragt und durch Falten dieses Überstandes die daraus entstehende Falz der Stützfolie als Abziehhilfe dient. Hierbei wird vollständig auf den Einsatz eines zusätzlichen Klebebandes als Abziehhilfe verzichtet. Stattdessen muss eine Stützfolie mit größeren Querabmessungen als das darunterliegende Folienlaminat aufgebracht werden, was grundsätzlich nachteilig ist, das das Erzeugen einer Schichtfolge mit gleichen Querabmessungen wesentlich einfacher z.B. mit Hilfe von Schablonen oder durch Zuschneiden des Laminats auf die gewünschte Breite erfolgen kann. Entsprechendes gilt für US 4 619 253. Auch hier wird die Abziehhilfe dadurch bereitgestellt, indem eine Stützfolie mit größeren Querabmessungen vorbereitet wird, die dann an beiden Ränder über darunterliegende Laminat übersteht.

US 2004/059273 A1 beschreibt ein Folienlaminatpflaster nach dem Oberbegriff von Anspruch 1. Die Abziehhilfe ist hierbei so um den Rand der Stützfolie, welcher über die Polymerfolie vorsteht, umgebogen, dass sie sowohl von oben auf die Stützfolie geklebt wird als auch von unten. Der eingeschlagene Rand der Abziehhilfe, der von unten an die Stützfolie geklebt ist, wird seinerseits von unten durch die Schutzabdeckung eingefasst. Das Aufkleben und Umlegen und anschließende unterseitige Ankleben der Abziehhilfe muss als weiterer komplizierter Verfahrensschitt in den Fertigungsprozeß des Folienlaminatpflasters integriert werden. Ein nachträgliche Aufkleben einer separat vorbereiteten Abziehhilfe aus gefaltetem und verklebtem Klebeband auf die Schutzabdeckung des Folienlaminats ist somit grundsätzlich nicht möglich. Dieses Folienlaminatpfaster ist wegen seiner Aufklebung der Abziehilfe auf beiden Seiten der Stützfolie kompliziert und fehleranfällig.

Es ist Aufgabe der vorliegenden Erfindung, ein konstruktiv einfaches, und damit einfach herstellbares Folienlaminatpflaster vorzuschlagen, das sich gleichwohl bei der Applikation einfach handhaben lässt.

Zur Lösung dieser Aufgabe dienen die kennzeichnenden Merkmale des Patentanspruchs 1 in Verbindung mit dessen Oberbegriff. Vorteilhafte Ausführungsformen der Erfindung sind in den Unternansprüchen angegeben. Ein Verfahren zur Herstellung eines solchen Folienlaminatpflasters ist in Anspruch 5 definiert.

Gemäß der vorliegenden Erfindung ist eine Abziehhilfe vorgesehen, die aus einem üblichen Klebeband durch Falzen entlang einer Linie parallel zur Längsrichtung des Klebebandes, aber gegenüber der Mittellinie des Klebebandes verschoben und durch Übereinanderlegen und Verkleben des Klebebandes gebildet ist, wobei die Abziehhilfe mit dem überstehenden streifenförmigen Klebebandbereich am Rand der Stützfolie aufgeklebt ist. Die Abziehhilfe kann so an dem durch Übereinanderlegen der Klebebandbereiche gebildeten Klebeband gegriffen werden, um so die Stützfolie abzuziehen.

Dabei ist das gefaltete Klebeband so auf die Stützfolie aufgeklebt, dass der nach der Faltung überstehende Klebebandstreifen vollflächig auf einem Randstreifen der Stützfolie aufgeklebt ist, während der übereinandergefaltete Bereich des Klebebandes über den Rand der Stützfolie vorsteht.

Vorzugsweise ist nur an einem Rand der Stützfolie die aus gefaltetem Kleberand gebildete Abziehhilfe angebracht.

Die Schutzabdeckung ist vorzugsweise durch eine klebstoffabweisende Trennpapierschicht gebildet, die durch eine Trennlinie unterteilt ist, so dass durch Krümmen des Folienlaminatpflasters entlang der Trennlinie das Abziehen der beiden durch die Trennlinie getrennten Trennpapierabschnitte erleichtert ist.

Vorzugsweise ist die Abziehhilfe aus gefaltetem Klebeband entlang des Randes der Stützfolie über die gesamte Länge des Folienlaminatpflasters aufgebracht.

Ferner ist ein Verfahren zur Herstellung eines Folienlaminatpflasters vorgesehen, bei dem eine Polymerfolie durch Extrusion oder Beschichtung auf einer Stützfolie gebildet oder eine Polymerfolie auf einer Seite mit einer Stützfolie versehen und auf ihrer anderen Seite mit einer hautverträglichen Klebeschicht versehen wird, und eine abziehbare Schutzabdeckung auf die Klebeschicht aufgebracht wird, und bei dem eine Abziehhilfe zum Abziehen der Stützfolie von der Polymerfolie aufgeklebt wird, dadurch gekennzeichnet, dass zum Anbringen der Abziehhilfe ein Klebeband bereitgestellt wird, das entlang einer Linie parallel zu seiner Längsrichtung so gefaltet und übereinandergelegt wird, dass ein Klebebandbereich als Randstreifen übersteht, und dieser überstehende Klebebandrandstreifen vollflächig auf einem Randstreifen der Stützfolie aufgeklebt wird, während der übereinandergefaltet und miteinander verklebte Bereich des Klebebandes über den Rand der Stützfolie vorsteht.

Die Vorteile des Verfahrens sind insbesondere, dass es kostengünstig durchführbar, technisch einfach und flexibel ist. Es werden nur wenige Ausgangsmaterialien benötigt, wobei dünnes, flexibles Klebeband einfach und in großer Vielfalt verfügbar ist. Ferner kann ein dünnes, flexibles Klebeband zur Bildung der Abziehhilfe verwendet werden, das durch die nach dem Umfalzen und Übereinanderlegen doppelte Klebebandanordung in diesem Anfassbereich größere Festigkeit hat, aber in dem überstehenden dünnen Klebebandrandstreifenbereich gute Klebrigkeit und Wickelbarkeit behält. Dadurch kann das Folienlaminatpflaster auch als Endlosprodukt in einer langen Bahn gefertigt und zunächst auf eine Rolle aufgewickelt werden. Wird in der bevorzugten Ausführungsform nur eine Abziehhilfe an einer Seite des Folienlaminatpflasters vorgesehen, so ergibt sich dadurch auch eine Ersparnis an Platz und Packmaterial gegenüber Folienlaminatpflastern mit an beiden Seiten überstehenden Abziehhilfen.

Die Erfindung wird im Folgenden anhand eines Ausführungsbeispiels in den Figuren beschrieben, die in
Figur 1: eine schematische Querschnittsansicht eines Folienlaminatpflasters und
Figur 2: eine schematische vergrößerte Querschnittsansicht einer Abziehhilfe zeigen.

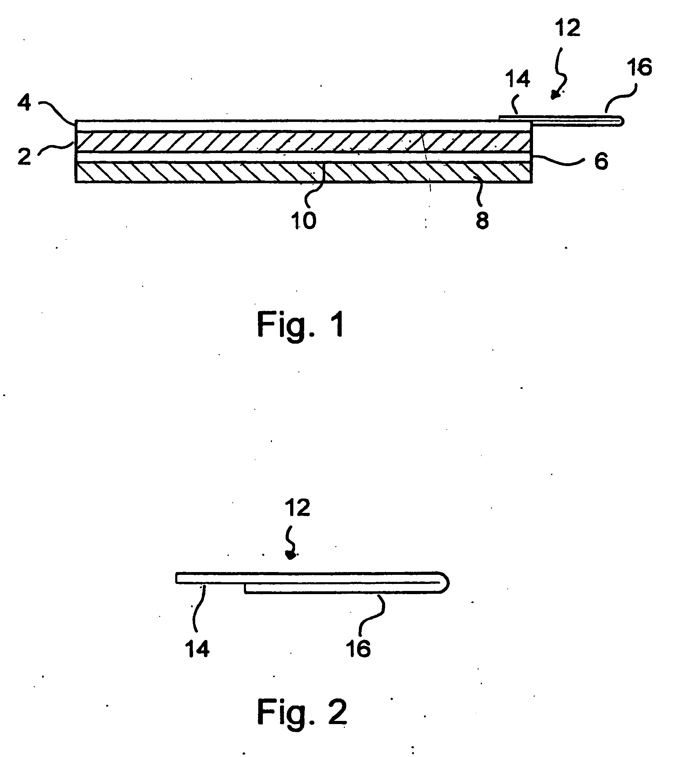

In der in Figur 1 dargestellten Querschnittsansicht des Folienlaminatsfplasters sind die einzelnen Lagen in ihren Dicken nicht maßstabsgerecht dargestellt; ferner sind die Dicken der Schichten im Vergleich zu ihrer Flächenausdehnung stark übertrieben, was auch für die Auschnittsvergrößerung von Figur 2 gilt.

Das Folienlaminatpflaster hat eine Polymerfolie 2, auf die eine Stützfolie 4 auflaminiert ist. Auf der gegenüberliegenden Seite der Polymerfolie 2 ist eine Klebstoffschicht 6 aufgebracht, die wiederum von einer Schutzabdeckung 8 abgedeckt ist. Die Schutzabdeckung 8 ist etwa in der Mitte mit einer Trennlinie 10 versehen. Durch Biegen des Folienlaminatpflasters entlang der Trennlinie 10 können so die beiden Teile der Schutzabdeckung 8 leicht von der Klebstoffschicht 6 angehoben und abgezogen werden, wenn das Folienlaminatpflaster appliziert werden soll.

Die Abziehhilfe 12, deren Querschnitt größer in Figur 2 gezeigt ist, besteht aus einem Klebeband, das entlang einer Linie parallel zu seiner Längsachse gefalzt und umgefaltet ist, wobei die Faltlinie aber gegenüber der Mittellinie des Klebebandes versetzt ist, so dass neben einem übereinandergelegten, doppelten Klebebandbereich 16 ein einfacher Klebebandrandstreifen 14 übrigbleibt. Mit diesem überstehenden Klebebandrandstreifen 14 ist das übereinandergefaltete Klebeband auf den Rand der Stützfolie 4 aufgeklebt. In dem in Figur 2 dargestellten Ausführungsbeispiel verhalten sich die Breiten des überstehenden einfachen Klebebandrandstreifens 14 und des übereinandergefalteten Doppelklebebandbereichs 16 etwa wie 1:2.

Bei der Herstellung kann eine Langrolle eines Folienlaminatprodukts abgewickelt werden. Auf die Seite der Stützfolie wird Klebeband aufgebracht, das vor dem Aufkleben auf die Stützfolie auf sich selbst gefaltet wurde, so dass es teilweise überlappt und eine freier Klebebandrandstreifen übersteht. Tesa 4150 hat sich als geeignetes Klebeband erwiesen. Nach Aufkleben des Klebebandranstreifens auf die Stützfolie wird das Produkt auf gewünschte Länge geschnitten und verpackt.

Das Folienlaminatpflaster kann z.B. mit 5, 10 oder 15 cm Breite und mit Längen von bis zu 10 m hergestellt werden und anschließend auf Rollen aufgerollt werden. Von der gelieferten Rolle kann der Anwender dann gewünschte Längen abschneiden. In anderen Ausführungsformen können vorgefertigte Abschnitte, z.B. mit Maßen von 10 cm x 8 cm bis 35 cm x 10 cm hergestellt werden. Diese vorgefertigten Abschnitte können dann auch mittig mit Wundvliesen versehen werden und beim Hersteller eingesiegelt und sterilisiert werden.

## Patentansprüche

1. Folienlaminatpflaster mit einer Polymerfolie, auf deren einer Seite eine abziehbare Stützfolie vorgesehen ist und deren andere Seite mit einer hautverträglichen Klebeschicht versehen ist, die wiederum von einer abziehbaren Schutzabdeckung abgedeckt ist, wobei auf die Stützfolie eine mit den Fingern greifbare Abziehhilfe zum Abziehen der Stützfolie von der Polymerfolie aufgeklebt ist, wobei die Abziehhilfe aus einem Klebeband (12) gebildet ist, **dadurch gekennzeichnet, dass** das Klebeband entlang einer Linie parallel zu seiner Längsrichtung so gefaltet und auf einem Teil seiner Fläche übereinandergelegt und verklebt ist, dass ein einfacher und somit einseitig klebender Klebebandrandstreifen (14) übersteht, und dieser überstehende Klebebandrandstreifen (14) vollflächig auf einem Randstreifen der Stützfolie (4) aufgeklebt ist, während der übereinandergefaltete und miteinander verklebte Bereich (16) des Klebebandes über den Rand der Stützfolie (4) vorsteht.

2. Folienlaminatpflaster nach Anspruch 1, **dadurch gekennzeichnet, dass** nur an einem Rand der Stützfolie (4) eine aus einem gefalteten Klebeband (12) gebildete Abziehhilfe angebracht ist.

3. Folienlaminatpflaster nach einem der vorhergehenden Ansprüche, **dadurch gekennzeichnet, dass** die Schutzabdeckung durch eine klebstoffabweisende Trennpapierschicht gebildet ist, wobei die Trennpapierschicht durch eine Trennlinie geteilt ist, so dass durch Krümmen des Folienlaminatpflasters entlang der Trennlinie das Abziehen der beiden durch die Trennlinie geteilten Trennpapierschichtbereiche erleichtert ist.

4. Folienlaminatpflaster nach einem der vorhergehenden Ansprüche, **dadurch gekennzeichnet, dass** das gefaltete Klebeband entlang des Randes der Stützfolie über die gesamte Länge des Folienlaminatpflasters angebracht ist.

5. Verfahren zur Herstellung eines Folienlaminatpflasters, bei dem eine Polymerfolie durch Extrusion oder Beschichten auf einer Stützfolie gebildet wird oder eine Polymerfolie auf einer Seite mit einer Stützfolie versehen wird und bei dem die Polymerfolie auf ihrer von der Stützfolie abgewandten Seite mit einer hautverträglichen Klebeschicht versehen wird, und eine abziehbare Schutzabdeckung auf die Klebeschicht aufgebracht wird, und bei dem eine Abziehhilfe zum Abziehen der Stützfolie von der Polymerfolie aufgeklebt wird, wobei zum Anbringen der Abziehhilfe ein Klebeband bereitgestellt wird, **dadurch gekennzeichnet, dass** das Klebeband entlang einer Linie parallel zu seiner Längsrichtung so gefaltet und übereinandergelegt und verklebt wird, dass ein einfacher und somit einseitig klebender Klebebandrandstreifen (14) übersteht und dieser überstehende Klebebandrandstreifen (14) vollflächig auf einem Randstreifen der Stützfolie (4) aufgeklebt wird, während der übereinandergefaltete und miteinander verklebte Bereich (16) des Klebebandes über den Rand der Stützfolie (4) vorsteht.

6. Verfahren nach Anspruch 5, **dadurch gekennzeichnet, dass** das Folienlaminatpflaster als streifenförmiges Endlosprodukt gefertigt und der umgefaltete Klebebandstreifen entlang eines Randes des streifenförmigen Endlosproduktes aufgeklebt wird.

## Claims

1. Film laminate plaster having a polymer film which on one side is provided with a peelable backing film and which on the other side is provided with a skin-friendly adhesive layer, which adhesive layer in turn is covered by a protective covering, wherein a peel-off aid is affixed to the backing film, which peel-off aid may be grasped with the fingers to peel the backing film off the polymer film, wherein the peel-off aid is formed from an adhesive tape (12), **characterised in that** the adhesive tape is folded along a line parallel to its longitudinal direction and folded over for a part of its surface area and sticked together such that a single and thus on one side adhesive edge stripe (14) of the adhesive tape is protruding, and **in that** that this protruding adhesive tape edge stripe (14) is with its entire area affixed to an edge stripe of the backing film (4), while the folded over and sticked together portion (16) of the adhesive tape is protruding over the edge of the backing film (4).

2. Film laminate plaster according to claim 1, **characterised in that** a peel-off aid formed of a folded over adhesive tape is affixed to one edge of the backing film (4) only.

3. Film laminate plaster according to any of the preceding claims, **characterised in that** the protective covering is formed of an adhesive-repellent separating paper sheet, said separating paper sheet being subdivided by a separating line such that by bending the film laminate plaster along the separating line the peeling off of the two separating paper sheet portions separated by the separating line is alleviated.

4. Film laminate plaster according to any of the preceding claims, **characterised in that** the folded up adhesive tape is affixed to the edge of the backing film over the entire length of the foil laminate plaster.

5. Method for manufacturing a foil laminate plaster, in which a polymer film is formed by extrusion or coating on a backing film or in which a polymer film is provided on one side with a backing film, the polymer film is provided with a skin-friendly adhesive layer on the side opposite to the backing film, and a peelable protective covering is applied to the adhesive layer, and a peel-off aid for peeling-off the backing film from the polymer film is affixed, wherein for affixing the peel-off aid an adhesive tape is provided, **characterised in that** the adhesive tape is folded over along a line parallel to its longitudinal direction and is folded together and sticked together, such that a single and thus on one side adhesive edge stripe (14) of the adhesive tape is protruding, and that this protruding adhesive tape edge stripe (14) is with its entire surface affixed to an edge stripe of the backing film (4) while the folded over and sticked together part (16) of the adhesive tape is protruding over the edge of the backing film (4).

6. Method according to claim 5, **characterised in that** the film laminate plaster is fabricated as a stripe-shaped, endless product and that the folded over adhesive tape stripe is affixed along an edge of the stripe-shaped, endless product.

## Revendications

1. Pansement stratifié avec un film de polymère dont un côté est muni d'un film de support amovible et l'autre côté est muni d'une couche adhésive hypoallergénique, elle-même recouverte d'un revêtement de protection amovible, moyennant quoi, sur le film de support, est collée une languette permettant de décoller le film de support du film de polymère, moyennant quoi la languette est constituée d'une bande adhésive (12), **caractérisé en ce que** la bande adhésive est pliée le long d'une ligne parallèle à sa direction longitudinale et superposée et collée seulement sur une partie de sa surface de telle sorte qu'une bordure de bande adhésive (14) simple, et donc adhésive d'un seul côté, dépasse et que cette bordure de bande adhésive (14) soit collée, sur toute sa surface, sur une bordure du film de support (4), tandis que la partie collée sur elle-même (16) de la bande adhésive dépasse du bord du film dé support (4).

2. Pansement stratifié selon la revendication 1, **caractérisé en ce qu'**une seule languette constituée d'une bande adhésive (12) pliée se trouve au bord du film de support (4).

3. Pansement stratifié selon l'une des revendications précédentes, **caractérisé en ce que** le revêtement de protection est constituée d'une couche de séparation anti-adhésive en papier, moyennant quoi la couche de séparation en papier est divisée par une ligne de séparation, de façon à ce que, grâce à une courbure du pansement stratifié le long de la ligne de séparation, le retrait des deux parties de la couche de séparation en papier divisées par la ligne de séparation soit facilitée.

4. Pansement stratifié selon l'une des revendications précédentes, **caractérisé en ce que** la bande adhésive pliée est appliquée le long du bord du film de support sur toute la longueur du pansement stratifié.

5. Procédé de fabrication d'un pansement stratifié dans lequel un film polymère est formé par extrusion ou revêtement sur un film de support ou bien un film polymère est muni, sur un côté, d'un film de support et dans lequel le film polymère est muni, sur le côté opposé au film support, d'une couche adhésive hypoallergénique, et un revêtement de protection amovible est appliqué sur la couche adhésive, et dans lequel une languette permettant la séparation du film support et du film polymère est collée, moyennant quoi, pour l'application de la languette, une bande adhésive est fabriquée, **caractérisé en ce que** la bande adhésive est pliée le long d'une ligne parallèle à sa direction longitudinale puis superposée et collée de façon à ce qu'une bordure de bande adhésive (14) simple, et donc adhésive d'un seul côté, dépasse et que cette bordure de bande adhésive (14) soit collée sur toute sa surface sur un bord du film de support (4) tandis que la partie repliée et collée sur elle-même (16) de la bande adhésive dépasse du bord du film de support (4).

6. Procédé selon la revendication 5, **caractérisé en ce que** le pansement stratifié est fabriqué sous la forme d'une bande continue et la bande adhésive repliée est collée le long du bord de la bande continue.
